# EUROPÄISCHE PATENTSCHRIFT

(11) **EP 1 089 818 B1**
(45) Veröffentlichungstag und Bekanntmachung des Hinweises auf die Patenterteilung: **06.04.2005**
(21) Anmeldenummer: 99931105.3
(22) Anmeldetag: 18.06.1999
(51) Int. Cl.: B01J 31/18, B01J 31/28, C07C 45/50, C07C 253/10

(54) **KATALYSATOR, UMFASSEND EINEN KOMPLEX EINES METALLS DER VIII. NEBENGRUPPE AUF BASIS EINES PHOSPHINITLIGANDEN, VERFAHREN ZUR HYDROFORMYLIERUNG**
CATALYST COMPRISING A COMPLEX OF A METAL OF GROUP VIII AND A PHOSPHINITE LIGAND, AND A METHOD FOR HYDROFORMYLATION
CATALYSEUR COMPRENANT UN COMPLEXE D'UN METAL DU GROUPE VIII ET D'UN LIGAND PHOSPHINITE, ET PROCEDE D'HYDROFORMYLATION

(30) Priorität: 18.06.1998 DE 19827232
(43) Veröffentlichungstag der Anmeldung: 11.04.2001
(73) Patentinhaber: BASF AKTIENGESELLSCHAFT, 67056 Ludwigshafen (DE)
(72) Erfinder: MAAS, Heiko, D-67105 Schifferstadt (DE); PACIELLO, Rocco, D-67098 Bad Dürkheim (DE); RÖPER, Michael, D-67157 Wachenheim (DE); FISCHER, Jakob, D-85414 Kirchdorf (DE)
(74) Vertreter: Kinzebach, Werner, Dr.
(86) Internationale Anmeldenummer: PCT/EP1999/004243
(87) Internationale Veröffentlichungsnummer: WO 1999/065606

(56) Entgegenhaltungen:
- EP-A- 0 614 870
- DE-A- 19 521 339
- US-A- 4 755 624
- US-A- 5 312 996

## Beschreibung

Die vorliegende Erfindung betrifft einen Katalysator, der wenigstens einen Komplex eines Metalls der VIII. Nebengruppe umfasst, welcher mindestens einen ein-, zwei- oder mehrzähnigen Phosphinitliganden umfasst, worin das Phosphor- und das Sauerstoffatom der Phosphinitgruppe Teil eines 5- bis 8-gliedrigen Heterocyclus sind, sowie ein Verfahren zur Hydroformylierung von Verbindungen, die wenigstens eine ethylenisch ungesättigte Doppelbindung enthalten, in Gegenwart eines solchen Katalysators.

Die Hydroformylierung oder Oxo-Synthese ist ein wichtiges großtechnisches Verfahren und dient der Herstellung von Aldehyden aus Olefinen, Kohlenmonoxid und Wasserstoff. Diese Aldehyde können gegebenenfalls im gleichen Arbeitsgang mit Wasserstoff zu den entsprechenden Oxo-Alkoholen hydriert werden. Die Reaktion selbst ist stark exotherm und läuft im Allgemeinen unter erhöhtem Druck und bei erhöhten Temperaturen in Gegenwart von Katalysatoren ab. Als Katalysatoren werden Co-, Rh- oder Ru-Verbindungen bzw. -komplexe eingesetzt, die zur Aktivitäts- und/oder Selektivitätsbeeinflussung mit amin- oder phosphinhaltigen Liganden modifiziert sein können. Zusätzliche Promotoren haben in der Praxis bisher keine Bedeutung erlangt. Bei der Hydroformylierungsreaktion kommt es aufgrund der möglichen CO-Anlagerung an jedes der beiden C-Atome einer Doppelbindung zur Bildung von Gemischen isomerer Aldehyde. Zusätzlich kann es beim Einsatz von internen Olefinen zu einer Doppelbindungsisomerisierung aus einer internen in Richtung auf eine terminale Position kommen. In diesen isomeren Gemischen ist der n-Aldehyd im Allgemeinen gegenüber dem iso-Aldehyd begünstigt, wobei jedoch aufgrund der wesentlich größeren technischen Bedeutung der n-Aldehyde eine Optimierung der Hydroformylierungskatalysatoren zur Erzielung einer größeren n-Selektivität angestrebt wird.

Eine Übersicht über Verfahren zur Hydroformylierung wird in Beller et al., Journal of Molecular Catalysis A, 104 (1995), Seiten 17-85, gegeben. Keine der dort genannten Literaturstellen beschreibt Hydroformylierungskatalysatoren auf Basis von zwei- oder mehrzähnigen Phosphinitliganden, wobei die Phosphinitgruppe Teil eines 5- bis 8-gliedrigen Heterocyclus ist.

Die EP-A 0 599 284 beschreibt Cycloolefinpolymer-Formmassen, die mit einer phosphororganischen Verbindung gegen unerwünschte, oxidative, thermische und photochemische Schädigung stabilisiert werden. Dabei können unter anderem auch Phosphinite, worin der Phosphor und das Sauerstoffatom der Phosphinitgruppe Teil eines 6-gliedrigen Heterocyclus sind, als Stabilisatoren eingesetzt werden. Die DE-A 40 21 195 und die WO 92/00306 beschreiben ein Verfahren zur Herstellung dieser phosphororganischen Verbindungen mittels Grignard-Synthese sowie deren Verwendung zur Stabilisierung von Kunststoffen. Komplexverbindungen dieser phosphororganischen Verbindungen und deren Verwendung als Liganden in Hydroformylierungskatalysatoren sind nicht beschrieben.

Die katalytische Hydrocyanierung zur Herstellung von Nitrilen aus Olefinen besitzt ebenfalls große technische Bedeutung. Dabei werden im Allgemeinen Mono-, Di- oder Polyolefine in Gegenwart von geeigneten Katalysatoren mit Cyanwasserstoff zu Mono-, Di- und Polynitrilen, bzw. Gemischen davon, umgesetzt, die insbesondere als Aminvorstufen, z. B. für die Herstellung von Polyamiden, überragende Bedeutung besitzen.

In "Applied Homogeneous Catalysis with Organometalic Compounds", Bd. 1, VCH Weinheim, S. 465 ff. wird allgemein die heterogen und homogen katalysierte Addition von Cyanwasserstoff an Olefine beschrieben. Dabei werden vor allem Katalysatoren auf Basis von Phosphin-, Phosphit- und Phosphonit-Komplexen des Nickels und Palladiums verwendet.

Übliche Katalysatoren für die Hydrocyanierung sind vor allem die bereits genannten Nickel(0)-Phosphitkatalysatoren.

C. A. Tolman et al. beschreiben in Organometallics 1984, 3, S. 33 ff. die katalytische Hydrocyanierung von Olefinen in Gegenwart von Nickel(0)-Phosphitkomplexen unter spezieller Berücksichtigung der Effekte von Lewis-Säuren auf die Cyanwasserstoffaddition.

In Advances in Catalysis, Band 33, 1985, Academic Press Inc., S. 1 ff. wird übersichtsartig die homogen Nickel-katalysierte Hydrocyanierung von Olefinen beschrieben. Als Katalysatoren werden Nickel(0)-Komplexe mit Phosphin- und Phosphitliganden eingesetzt.

In J. Chem. Soc., Chem. Commun., 1991, S. 1292, werden chirale Aryldiphosphite als Liganden für Hydrocyanierungskatalysatoren beschrieben. Bei diesen Liganden ist die Phosphitgruppe über zwei ihrer Sauerstoffatome an die 3- und 3'-Positionen einer 2,2'-Binaphthyleinheit gebunden, mit der sie so einen 7-gliedrigen Heterocyclus bildet. Zusätzlich können zwei dieser Heterocyclen ebenfalls über eine 2,2'-Binaphthyleinheit zu einem zweizähnigen Chelatliganden verknüpft sein.

In J. Chem. Soc., Chem. Commun., 1991, S. 803 ff., werden dazu analoge Chelatdiphosphit-Komplexe von Nickel(0) und Platin(0) beschrieben, wobei anstelle einer 2,2'-Binaphthyleinheit eine 2,2'-Biphenyleinheit eingesetzt wird.

Die WO 95/28228 beschreibt ein Verfahren zur Hydrocyanierung von aliphatischen Monoolefinen, die gegebenenfalls zusätzlich eine nichtkonjugierte Nitrilgruppe oder eine nichtkonjugierte oder konjugierte Estergruppe aufweisen. Die eingesetzten Nickel(0)-Katalysatoren umfassen dabei ebenfalls zweizähnige Phosphitliganden, bei denen die Phosphitgruppen Teile von Aryl-anellierten Heterocyclen sind.

Die WO 95/29153 beschreibt ein verfahren zur Hydrocyanierung von Monoolefinen, wobei Katalysatoren auf Basis von nullwertigem Nikkel und einzähnigen Phosphitliganden eingesetzt werden. Bei diesen Liganden ist die Phosphitgruppe wiederum zusammen mit zwei ihrer Sauerstoffatome Teil eines Aryl-annellierten 7-gliedrigen Heterocyclus. Das dritte Sauerstoffatom der Phosphitgruppe trägt einen t.-Butyl-substituierten Phenylrest, welcher gegebenenfalls noch weitere Substituenten aufweisen kann.

Die WO 96/11182 beschreibt ein verfahren zur Hydrocyanierung von aliphatischen, monoethylenisch ungesättigten Verbindungen, bei denen die ethylenische Doppelbindung nicht in Konjugation zu einer anderen ungesättigten Gruppe steht oder bei denen die ethylenische Doppelbindung in Konjugation zu einer Estergruppe steht. Dabei wird ein Nickel(0)-Katalysator auf Basis eines mehrzähnigen Phosphitliganden in Gegenwart einer Lewis-Säure als Promotor eingesetzt. Die Phosphitgruppen dieser mehrzähnigen Liganden sind dabei wiederum Bestandteile von Aryl-annellierten Heterocyclen und ggf. über Aryl-anellierte Gruppen verbrückt.

Die WO 96/22968 beschreibt ein Verfahren zur Hydrocyanierung diolefinischer Verbindungen und zur Isomerisierung der resultierenden, nichtkonjugierten 2-Alkyl-3-monoalkennitrile durch Umsetzung eines acyclischen, aliphatischen Diolefins mit einer Cyanwasserstoffquelle. Die Umsetzung findet dabei in der flüssigen Phase statt. Es werden Hydrocyanierungskatalysatoren eingesetzt, die denen in der wo 96/11182 beschriebenen analog sind.

Die US-A 5,512,695 hat einen der WO 95/28228 entsprechenden Offenbarungsgehalt.

Neben den zuvor beschriebenen Hydrocyanierungskatalysatoren auf Basis von ein-, zwei- und mehrzähnigen Phosphitliganden sind auch Katalysatoren auf Basis von Phosphinitliganden bekannt. In J. Am. Chem. Soc., 1994, 116, S. 9869 f. und in der US-A-5,484,902 sind Katalysatoren für die enantioselektive Hydrocyanierung aromatischer Vinylverbindungen auf der Basis von einem chiralen, nichtracemischen, zweizähnigen Chelatphosphinitliganden beschrieben. Als Ligand wird dabei vorzugsweise ein Phenyl-2,3-bis-O-(3,5-bis-(trifluormethyl)phenyl)phosphino-4,6-O-benzyliden-β-D-glucopyranosid eingesetzt.

Die US-A 5,523,453 beschreibt ein Verfahren zur Hydrocyanierung von Monoolefinen, welche gegebenenfalls zusätzlich eine Cyanogruppe aufweisen können, in Gegenwart einer Lewis-Säure als Promotor und einem Nickel(0)-Katalysator. Diese Katalysatoren weisen Liganden auf Basis von Chelatphosphiniten auf, bei denen zwei arylsubstituierte Phosphinitgruppen über ihr Sauerstoffatom und eine Aryl-anellierte Alkylenbrücke miteinander verknüpft sind.

Keine der zuvor genannten Literaturstellen beschreibt Hydrocyanierungskatalysatoren auf Basis von Phosphinitliganden, worin die Phosphinitgruppe Teil eines 5- bis 8-gliedrigen Heterocyclus ist.

Die US-A 3,766,237 beschreibt ein Verfahren zur Hydrocyanierung ethylenisch ungesättigter Verbindungen, welche weitere funktionelle Gruppen, wie z. B. Nitrile, aufweisen können, in Gegenwart eines Nickelkatalysators. Diese Nickelkatalysatoren tragen vier Liganden der allgemeinen Formel M(X,Y,Z), wobei X, Y und Z unabhängig voneinander für einen Rest R oder OR stehen und R ausgewählt ist unter Alkyl- und Arylgruppen mit bis zu 18 Kohlenstoffatomen. Dabei werden jedoch nur Phosphine und Phosphite explizit genannt und in den Beispielen für die Hydrocyanierung eingesetzt. Dagegen ist nicht offenbart, dass Phosphinite, bei denen die Phosphinitgruppe Teil eines Heterocyclus ist, als Liganden für Nickel(0)-Hydrocyanierungskatalysatoren eingesetzt werden können.

Die US 5,312,996 beschreibt ein Hydroformylierungsverfahren zur Herstellung von 1,6-Hexandiol aus Butadien in Gegenwart eines Rhodiumkomplexes mit einem Polyphosphitliganden.

Die EP-A-0 614 870 beschreibt ein Verfahren zur Herstellung optisch aktiver Aldehyde in Gegenwart eines Rhodium-Phosphin-Komplexes als Katalysator, der einen Liganden mit Binaphthyl-Rückgrat aufweist, an den eine Phosphingruppe und eine Phosphitgruppe gebunden sind.

Die WO 99/13983 beschreibt einen Katalysator, der wenigstens einen Nickel(0) komplex auf Basis eines Phosphinitliganden umfasst und dessen Verwendung zur Hydrocyanierung.

Der vorliegenden Erfindung liegt die Aufgabe zugrunde, neue Katalysatoren auf Basis von Komplexen eines Metalls der VIII. Nebengruppe zur Verfügung zu stellen. Diese sollen sich vorzugsweise zur Hydroformylierung eignen und eine gute katalytische Aktivität aufweisen.

Überraschenderweise wurden nun Katalysatoren auf Basis von Komplexen eines Metalls der VIII. Nebengruppe gefunden, welche mindestens einen ein-, zwei- oder mehrzähnigen Phosphinitliganden umfassen, wobei die Phosphinitgruppe Teil eines 5- bis 8-gliedrigen Heterocyclus ist.

Gegenstand der vorliegenden Erfindung ist somit ein Katalysator, umfassend wenigstens einen Komplex eines Metalls der VIII. Nebengruppe mit mindestens einem ein-, zwei- oder mehrzähnigen Phosphinitliganden der allgemeinen Formel I 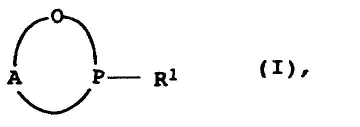 worin
- A: für einen 2,2'-Biphenylylen-, 2,2'-Binaphthylylen- oder 2,3-Xylylen-Rest steht, der 1, 2 oder 3 Substituenten, ausgewählt unter Alkyl, Alkoxy oder Halogen, tragen kann,
- R¹: für Alkyl, Aryl oder Hetaryl steht, welche gegebenenfalls einen, zwei oder drei Substituenten, ausgewählt unter Alkyl, Cycloalkyl, Aryl, Alkoxy, Cycloalkyloxy, Aryloxy, Halogen, Trifluormethyl, Nitro, Cyano, Carboxyl, Carboxylat, Acyl, Sulfonyl, NE¹E² und Alkylen-NE¹E² tragen, wobei E¹ und E² gleich oder verschieden sind und ausgewählt sind unter Alkyl, Cycloalkyl und Aryl; oder für einen Rest der allgemeinen Formel II

-X-Y (II)

steht, worin
X entweder für eine C₂- bis C₈-Alkylenbrücke steht, die gegebenenfalls durch SiR^{a}R^{b} , N, NR^{c}, O oder S unterbrochen ist, worin R^{a} und R^{b} für Wasserstoff, Alkyl, Alkoxy, Halogen, Trifluormethyl, Nitro oder Cyano stehen und R^{c} für Wasserstoff, Alkyl oder Aryl steht, wobei Aryl gegebenenfalls durch Alkyl, Alkoxy, Halogen, Trifluormethyl, Nitro oder Cyano ein- oder zweifach substituiert ist, gegebenenfalls eine, zwei oder drei Doppelbindungen aufweist und/oder gegebenenfalls ein-, zwei- oder dreifach mit Aryl und/oder Hetaryl anelliert ist, wobei die Aryl- oder Hetarylgruppen unabhängig voneinander gegebenenfalls einen, zwei, drei oder vier Substituenten ausgewählt unter Alkyl, Cycloalkyl, Aryl, Alkoxy, Cycloalkyloxy, Aryloxy, Halogen, Trifluormethyl, Nitro, Cyano, Acyl, Carboxyl, Carboxylat, Sulfonyl, Sulfonat, NE¹E² und Alkylen-NE¹E² tragen, wobei E¹ und E² die zuvor angegebenen Bedeutungen besitzen, und benachbarte anellierte Ringe gegebenenfalls über SiR^{a}R^{b} , N, NR^{c}, O oder S oder über eine C₁-C₆-Alkylenbrücke, die gegebenenfalls zusätzlich eine Gruppe, ausgewählt unter SiR^{a}R^{b} , N, NR^{c}, O oder S, trägt, verbunden sind, worin R^{a}, R^{b} und R^{c} wie o. a. definiert sind; oder
für eine gegebenenfalls substituierte Metallocenbrücke steht; und
Y für einen Rest der Formel III 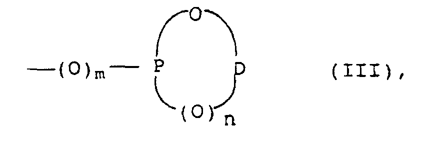 steht, worin
D die zuvor für A angegebenen Bedeutungen besitzt,und
m und n unabhängig voneinander für 0 oder 1 stehen,
oder ein Salz davon.

Im Rahmen der vorliegenden Erfindung umfasst der Ausdruck 'Alkyl' geradkettige und verzweigte Alkylgruppen. Vorzugsweise handelt es sich dabei um geradkettige oder verzweigte C₁-C₈-Alkyl-, bevorzugter C₁-C₆-Alkyl- und besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl.

Im Rahmen dieser Erfindung umfasst der Ausdruck "Alkylen" geradkettige, gegebenenfalls substituierte Alkylengruppen mit 1 bis 8 Kohlenstoffatomen. Falls X für eine Alkylenbrücke steht, so weist diese 2 bis 8, vorzugsweise 3 bis 6 und besonders bevorzugt 3 bis 5 Kohlenstoffatome auf. Die Alkylenbrücke kann durch Heterogruppen, z. B. Si, SiR^{a}R^{b}, N, NR^{c}, O oder S, insbesondere O und NR^{c}, unterbrochen sein, worin R^{a} und R^{b} für Wasserstoff, Alkyl, Alkoxy, Halogen, Trifluormethyl, Nitro oder Cyano stehen und R^{c} für Wasserstoff, Alkyl oder Aryl steht, wobei Aryl gegebenenfalls durch Alkyl, Alkoxy, Halogen, Trifluormethyl, Nitro oder Cyano ein- oder zweifach substituiert ist. Bevorzugt sind für X Alkylenbrücken, die keine Heteroatome aufweisen. Ferner kann die Alkylenbrücke eine, zwei oder drei, bevorzugt eine oder zwei, Doppelbindungen aufweisen und/oder gegebenenfalls ein-, zwei- oder dreifach, bevorzugt ein- oder zweifach, mit Aryl und/oder Hetaryl, vorzugsweise mit Aryl, anelliert sein kann. Gegebenfalls anellierte Aryl- oder Hetarylgruppen können unabhängig voneinander einen, zwei, drei oder vier, vorzugsweise einen, zwei oder drei Substituenten, ausgewählt unter Alkyl, Cycloalkyl, Aryl, Alkoxy, Cycloalkyloxy, Aryloxy, Halogen, Trifluormethyl, Nitro, Cyano, Acyl, Carboxyl, Carboxylat, NE¹E² oder Alkylen-NE¹E² tragen, wobei E¹ und E² gleich oder verschieden sind und ausgewählt sind unter Alkyl, Cycloalkyl und Aryl. Wenn eine Alkylengruppe eine NE¹E²-Gruppe trägt, weist sie vorzugsweise 1 bis 4 Kohlenstoffatome und besonders bevorzugt 1 oder 2 Kohlenstoffatome auf, ganz besonders bevorzugt sind Methylen und Ethylen. Vorzugsweise sind die Substituenten der anellierten Aryle und Hetaryle ausgewählt unter Alkyl, Alkoxy, Trifluormethyl, Halogen und Carboxylat.

Bei der Cycloalkylgruppe handelt es sich vorzugsweise um eine C₅-C₇-Cycloalkylgruppe, wie Cyclopentyl, Cyclohexyl oder Cycloheptyl.

Wenn die Cycloalkylgruppe substituiert ist, weist sie vorzugsweise 1, 2, 3, 4 oder 5, insbesondere 1, 2 oder 3 Substituenten, ausgewählt unter Alkyl, Alkoxy oder Halogen auf.

Aryl steht vorzugsweise für Phenyl, Tolyl, Xylyl, Mesityl, Naphthyl, Anthryl, Phenanthryl, Naphthacenyl und insbesondere für Phenyl oder Naphthyl.

Substituierte Arylreste weisen vorzugsweise 1, 2, 3 oder 4, insbesondere 1, 2 oder 3 Substituenten, ausgewählt unter Alkyl, Alkoxy, Carboxylat oder Halogen auf.

Hetaryl steht vorzugsweise für Pyridyl, Chinolinyl, Acridinyl, Pyridazinyl, Pyrimidinyl oder Pyrazinyl.

Substituierte Hetarylreste weisen vorzugsweise 1, 2 oder 3 Substituenten, ausgewählt unter Alkyl, Alkoxy oder Halogen auf.

Die obigen Ausführungen zu Alkyl-, Cycloalkyl- und Arylresten gelten entsprechend für Alkoxy-, Cycloalkyloxy- und Aryloxyreste.

Die Reste NE¹E² stehen vorzugsweise für N,N-Dimethyl-, N,N-Diethyl-, N,N-Dipropyl-, N,N-Diisopropyl-, N,N-Di-n-butyl-, N,N-Dit.-butyl-, N,N-Dicyclohexyl- oder N,N-Diphenyl-amino.

Halogen steht für Fluor, Chlor, Brom und Iod, bevorzugt für Fluor, Chlor und Brom.

Carboxylat steht im Rahmen dieser Erfindung vorzugsweise für ein Derivat einer Carbonsäurefunktion, insbesondere für ein Metallcarboxylat, eine Carbonsäureesterfunktion oder eine Carbonsäureamidfunktion, besonders bevorzugt für eine Carbonsäureesterfunktion.

Wenn R¹ für einen Rest der allgemeinen Formel II

-X-Y (II)

steht, so ist X vorzugsweise ausgewählt unter 2,3-xylylen, 2,2'-Biphenylylen, 2,2'-Binaphthylylen und durch O, S und NR^{c} verknüpften Arylen, insbesondere Phenylen und Naphthylen, wobei R^{c} die oben angegebene Bedeutung besitzt. Dabei kann jede Phenyleinheit zusätzlich 1, 2 oder 3 Substituenten, jede Naphthyleinheit 1 bis 4, vorzugsweis 1 bis 3 Substituenten, ausgewählt unter Alkyl, Cycloalkyl, Aryl, Alkyloxy, Halogen, Trifluormethyl, Nitro, Cyano, Acyl, Carboxyl und Carboxylat, insbesondere Alkyl, Alkoxy, Trifluormethyl und Carboxylat, aufweisen. Die Phenyl- bzw. Naphthyleinheiten können zusätzlich über O, S oder NR^{c} oder eine C₁bis C₆-Alkylenbrücke, die durch O, S oder NR^{c} unterbrochen sein kann, verknüpft sein, wobei R^{c} die bereits oben angegebene Bedeutung besitzt. Y ist vorzugsweise ein Phosphit-, Phosphonit- oder Phosphinit-Ligand, bei dem die O-P-O- (n=1) bzw. P-O-Gruppe (n=O) ebenfalls Teil eines 5- bis 8-gliedrigen Heterocyclus ist. Für diesen gelten die bereits für den Heterocyclus des erfindungsgemäßen Phosphinitliganden der Formel I dargelegten obigen Ausführungen. Insbesondere gelten für D die zuvor für A definierten Bedeutungen, wobei A und D gleich oder verschieden sein können.

X kann jedoch auch für eine Metallocenbrücke stehen, die gegebenenfalls einen oder mehrere weitere Substituenten aufweist.

Der Begriff "Metallocen" steht im Rahmen dieser Erfindung für einen Sandwich-Metallkomplex mit zwei η⁵- bzw. η⁶-gebundenen ungesättigten oder aromatischen Ringsystemen, die, abhängig vom zentralen Metallatom, parallel oder gewinkelt angeordnet sein können. Geeignet als Zentralmetallatom sind alle Metalle, die stabile Sandwichkomplexe bilden, die auch unter den Einsatzbedingungen der erfindungsgemäßen Katalysatoren hinreichende Stabilität aufweisen, insbesondere jedoch Fe, Co, Ni, Ru, Os, Rh, Mn, Cr oder V, besonders bevorzugt Fe, Co und Ni. Vorzugsweise weisen die Metalle in den Metallocenkomplexen die formalen Oxidationszahlen 0, +1 oder +2 auf. Geeignete η⁵- bzw. η⁶-komplexierbare ungesättigte oder aromatische Ringsysteme sind Cyclopentadienyl, Indenyl, Fluorenyl, Azulenyl, Benzol, Naphthalin, Anthracen und Phenanthren. Die genannten Ringsysteme können z. B. 1 bis 5, bevorzugt 1 bis 3 der vorgenannten Substituenten, insbesondere Alkyl, Alkoxy und Alkylen-NE¹E² und besonders bevorzugt Methyl, Isopropyl und tert.-Butyl, aufweisen.

Bevorzugt steht X für einen Rest der Formel IV.1, IV.2, IV.3, IV.4, IV.5, IV.6, IV.7 oder IV.8 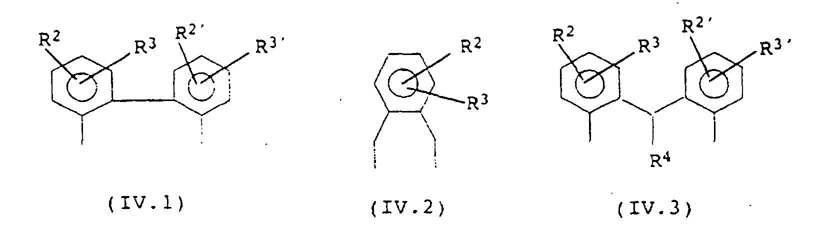 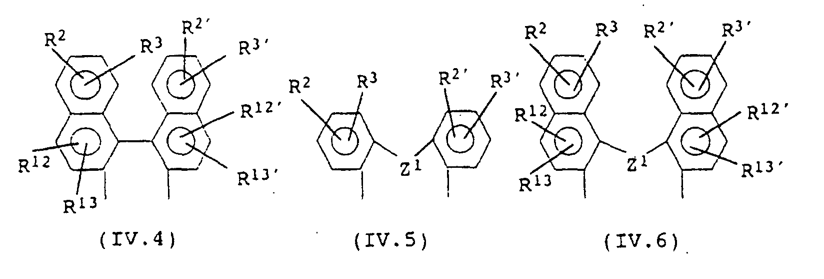 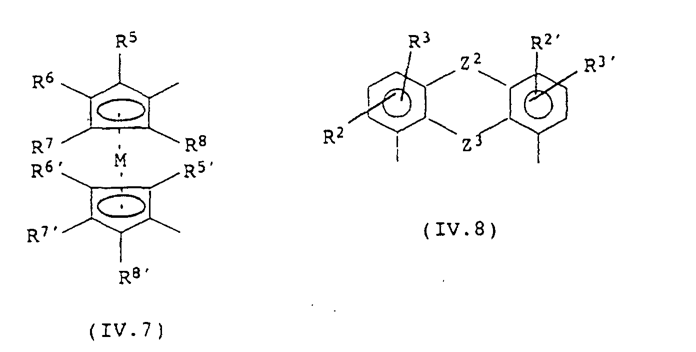 worin
- R² , R^{2'}, R³ und R^{3'}: unabhängig voneinander für wasserstoff, Alkyl, Alkoxy, Halogen, Trifluormethyl, Nitro, Cyano, Acyl, Carboxyl oder Carboxylat stehen,
- R⁴: für Wasserstoff, Alkyl, vorzugsweise Methyl, oder Aryl, vorzugsweise Phenyl, steht, welches gegebenenfalls durch Alkyl, Alkoxy, Halogen, Trifluormethyl, Nitro oder Cyano substituiert sein kann, und
- R⁵, R^{5'}, R⁶, R^{6'}, R⁷, R^{7'}, R⁸, R^{8'}, R¹², R^{12'}, R¹³ und R^{13'}: die für R² und R³ genannten Bedeutungen besitzen oder unabhängig voneinander ausgewählt sind unter Cycloalkyl, Aryl oder Alkyl, welches durch ein Sauerstoffatom unterbrochen oder mit einem Rest der Formel NE¹E² substituiert sein kann, wobei E¹ und E² die oben angegebene Bedeutung besitzen; oder jeweils zwei benachbarte Substituenten R⁵, R⁶, R⁷, R⁸ und/oder R^{5'} , R^{6'} , R^{7'} , R^{8'} mit dem sie verbindenden Teil des Cyclopentadienylrings für einen aromatischen oder nichtaromatischen 5- bis 7-gliedrigen Carbo- oder Heterocyclus stehen, wobei der Heterocyclus 1, 2 oder 3 Heterogruppen, ausgewählt unter O, N, NR^{c} und S, aufweist,
- M: für Fe, Co, Ni, Ru, Os, Rh, Mn, Cr oder V steht,
- Z¹: für O, S und NR^{c} oder für eine C₂- bis C₃-Alkylenbrücke, die durch O, S oder NR^{c} unterbrochen ist, steht,
- Z² und Z³: unabhängig voneinander für CH₂, SiR^{a}R^{b}, O, S, NR^{c} oder CR^{a}R^{b} stehen, wobei R^{a}, R^{b} und R^{c} jeweils die oben angegebene Bedeutung besitzen.

Die obigen Ausführungen zu bevorzugten Resten A gelten entsprechend für bevorzugte Reste D.

Wenn in IV.7 einer der Reste R⁵, R^{5'}, R⁶, R^{6'}, R⁷, R^{7'}, R⁸ oder R^{8'} für Alkyl steht, welches durch ein Sauerstoffatom in Etherbindung unterbrochen ist, so können sie z. B. für 2-Methoxyethyl, 2-Ethoxyethyl, 2-Propoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2-oder 4-Butoxybutyl stehen.

Wenn in IV.7 einer der Reste R⁵, R^{5'}, R⁶, R^{6'}, R⁷, R^{7'}, R⁸ oder R^{8'} für Alkyl steht, welches durch einen Rest der Formel NE¹E² substituiert ist, so können sie z. B. für N,N-Dimethyl-aminomethyl, N,N-Dimethyl-aminoethyl, N,N-Diethyl-aminomethyl, N,N-Diethylaminoethyl oder N,N-Dimethyl-aminopropyl stehen.

Wenn in IV.7 jeweils zwei der Substituenten R⁵, R⁶, R⁷, R⁸ und/oder R^{5'}, R^{6'}, R^{7'}, R^{8'} in benachbarten Positionen zusammen mit dem sie verbindenden Teil des Cyclopentadienylrings für einen aromatischen oder nichtaromatischen, 5- bis 7-gliedrigen Carbo- oder Heterocyclus stehen, der zusätzlich 1, 2 oder 3 Heteroatome, ausgewählt unter O, N und S, aufweist, so können sie z. B. für Indenyl, Fluorenyl, Azulenyl etc. stehen.

Die beiden Cyclopentadienylringe des Metallocens der Formel IV.7 können in ekliptischer oder gestaffelter Konformation mit variierenden Konformationswinkeln vorliegen. Die Ebenen der Cyclopentadienylringe können z. B. in Abhängigkeit vom zentralmetall, parallel oder gegeneinander geneigt sein.

Der Rest A steht erfindungsgemäß für einen 2,2'-Biphenylylen-, 2,2'-Binaphthylylen- oder 2,3-Xylylen-Rest, der 1, 2 oder 3 Substituenten, ausgewählt unter Alkyl, Alkoxy oder Halogen, tragen kann. Alkyl steht dabei vorzugsweise für C₁-C₄-Alkyl und insbesondere für t.-Butyl. Alkoxy steht dabei vorzugsweise für C₁-C₄-Alkoxy und insbesondere für Methoxy. Halogen steht insbesondere für Fluor, Chlor oder Brom. Die obigen Ausführungen zu bevorzugten Resten A gelten entsprechend für bevorzugte Reste D.

Nach einer weiteren bevorzugten Ausführungsform umfassen die erfindungsgemäßen Katalysatoren mindestens einen Phosphinitliganden der Formel I, wobei
- R¹: für Phenyl oder Naphthyl steht, welches einen, zwei oder drei der folgenden Substituenten: Alkyl, Alkoxy, Halogen, Nitro, Cyano, Carboxyl oder NE¹E² tragen kann, wobei E¹ und E² die zuvor angegebenen Bedeutungen besitzen, oder für einen Rest der Formel II steht, worin
X für eine C₄- bis C₅-Alkylenbrücke steht, welche eine oder zwei Doppelbindungen aufweisen und/oder ein- oder zweifach mit Aryl und/oder Hetaryl anelliert sein kann, wobei die Aryl- oder Hetarylgruppen einen, zwei oder drei der folgenden Substituenten: Alkyl, Cycloalkyl, Aryl, Alkoxy, Cycloalkyloxy, Aryloxy, Halogen, Nitro, Cyano, Carboxyl, Carboxylat, Alkylen-NE¹E² oder NE¹E² tragen können, wobei E¹ und E² die zuvor angegebenen Bedeutungen besitzen, und
Y für einen Rest der Formel III steht, worin D für einen Rest der Formeln IV.1, IV.2, IV.3, IV.4, IV.5, IV.6 oder IV.8 steht.

Wenn R¹ für Phenyl oder Naphthyl steht, so weist dieses vorzugsweise 1, 2 oder 3 Substituenten, ausgewählt unter C₁-C₄-Alkyl, C₁-C₄-Alkoxy oder Halogen auf. Insbesondere sind die Substituenten ausgewählt unter t.-Butyl, Methoxy, Trifluormethyl, Fluor, Chlor und Brom.

Wenn R¹ für einen Rest der Formel II steht, worin X für eine C₄-C₅-Alkylenbrücke steht, so ist diese vorzugsweise ein- oder zweifach mit Phenyl und/oder Naphthyl anelliert, wobei die Phenyl- oder Naphthylgruppen 1, 2 oder 3 der folgenden Substituenten: t.-Butyl, Methoxy, Carboxylat, Fluor, Chlor oder Brom aufweisen können.

Nach einer geeigneten Ausführungsform sind die Phosphinitliganden der Formel I ausgewählt unter Liganden der Formel Ia bis Ii 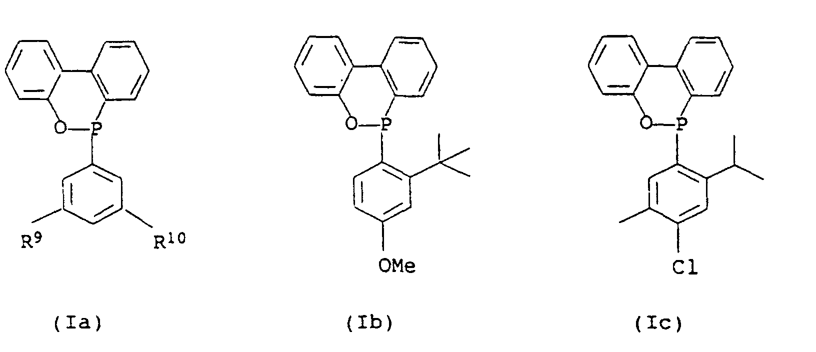 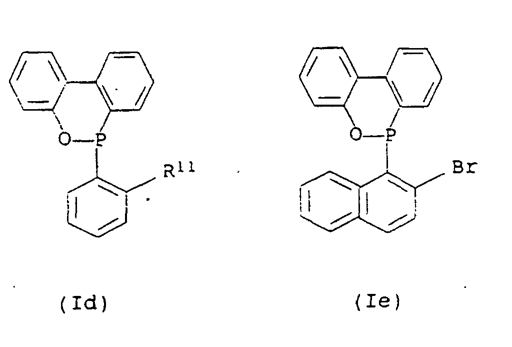 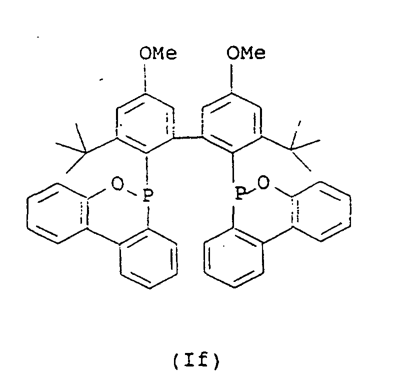 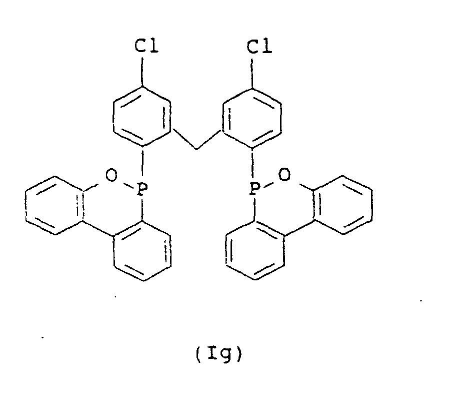 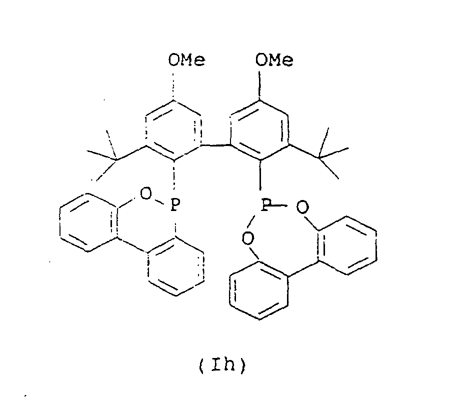 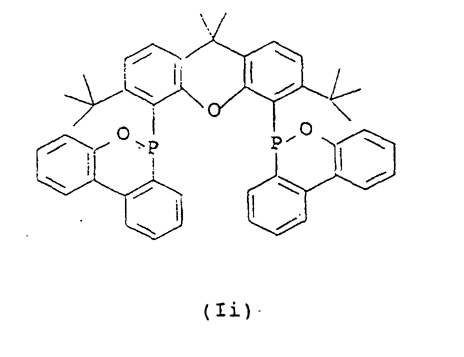 worin
- R⁹ und R¹⁰: unabhängig voneinander für Wasserstoff oder Trifluormethyl stehen,
- R¹¹: für Fluor oder Trifluormethyl steht.

Die erfindungsgemäßen Katalysatoren können einen oder mehrere der Phosphinitliganden der Formel I aufweisen. Zusätzlich zu den zuvor beschriebenen Liganden der allgemeinen Formel I können sie noch wenigstens einen weiteren Liganden, der ausgewählt ist unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃ sowie ein-, zwei- und mehrzähnigen Phosphin-, Phosphinit-, Phosphonit- und Phosphitliganden aufweisen. Diese weiteren Liganden können ebenfalls ein-, zwei- oder mehrzähnig sein und am Metallatom des Katalysatorkomplexes koordinieren. Geeignete weitere phosphorhaltige Liganden sind z. B. übliche Phosphin-, Phosphonit-, und Phosphitliganden.

Zur Herstellung der erfindungsgemäß eingesetzten Phosphinitliganden der Formel I kann man z. B. eine Hydroxylgruppen-haltige Verbindung der Formel V mit einem Phosphortrihalogenid, bevorzugt PCl₃, zu einer Verbindung der Formel VI umsetzen. Diese wird dann mit einer metallorganischen Verbindung der Formel R¹[M(Q)_{q}]ᵣ, umgesetzt. Diese wird aus R¹[(Q)_{q}]ᵣ hergestellt und als in situ generierte oder als zuvor isolierte Verbindung eingesetzt. Die Herstellung kann durch folgendes Schema veranschaulicht werden, 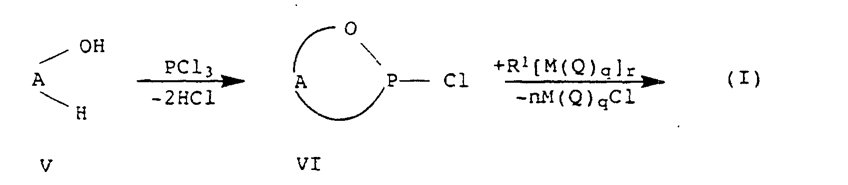 wobei A und R¹ die zuvor angegebenen Bedeutungen besitzen und M für Lithium oder Magnesium, X für H, Cl, Br, q für 0 oder 1 und r für 1 oder 2 steht.

Ein solches Verfahren zur Synthese von Phosphiniten wird in der WO 92/00306 beschrieben, worauf hiermit Bezug genommen wird.

Beispiele für geeignete Verbindungen der Formel V sind z. B. Biphenyl-2-ol, Binaphthyl-2-ol, 1,1'-Biphenyl-4-phenyl-2-ol, 1,1'-Biphenyl-3,3',5,5'-tetra-t-butyl-2-ol, 1,1'-Biphenyl-3,3'-di-t-amyl-5,5'-dimethoxy-2-ol, 1,1'-Biphenyl-3,3'-di-t-butyl-5,5'-dimethoxy-2-ol, 1,1'-Biphenyl-3,3'-di-t-butyl-2-ol, 1,1'-Biphenyl-3,3'-di-t-butyl-6,6'-dimethyl-2-ol, 1,1'-Biphenyl-3,3',5,5'-tetra-t-butyl-6,6'-dimethyl-2-ol, 1,1'-Biphenyl-3,3'di-t-butyl-5,5'-di-t-butoxy-2-ol, 1,1'-Biphenyl-3,3'-di-t-hexyl-5,5'-dimethoxy-2-ol, 1,1'-Biphenyl-3-t-butyl-5,5'-dimethoxy-2-ol, 1,1'-Biphenyl- 3,3'- di[2-(1,3-dioxacyclohexan)]-5,5'-dimethoxy-2-ol, 1,1'-Biphenyl- 3,3'-diformyl-5,5'-dimethoxy-2-ol und 1,1'-Biphenanthryl-2-ol, insbesondere Biphenyl-2-ol und Binaphthyl-2-ol.

Geeignete Verbindungen der Formel R¹[(Q)_{q}]ᵣ sind zum Beispiel unsubstituierte Aromaten (Q = H), wie Benzol, Naphthalin, Tetralin, Anthrazen oder Phenanthren, insbesondere Benzol und Naphthalin, monosubstituierte Aromaten, wie Halogenaromaten (Q = Cl, Br, I), zum Beispiel Halogenbenzol, Halogennaphthalin, insbesondere Chlorbenzol und Brombenzol und Monoalkyl- oder Monoarylaromaten, wobei Alkyl bzw. Aryl die bereits vorne definierte Bedeutung besitzen kann, aber insbesondere für C₁-C₈-Alkylreste bzw. unsubstituierte oder substituierte Phenylreste steht. Geeignete Monoalkylaromaten (Q = H) sind z. B. Toluol, Ethylbenzol, n-Propylbenzol, Cumol, n-Butylbenzol, sec-Butylbenzol und tert-Butylbenzol, insbesondere Toluol, Cumol und tert-Butylbenzol. Geeignete Monoarylaromaten sind z. B. Biphenyl und substituierte Biphenyle.

Ebenfalls geeignete Verbindungen der Formel R¹[(Q)_{q}]ᵣ sind mehrfach substituierte Aromaten, wie alkyl- bzw. arylsubstituierte Halogenaromaten, mehrfach alkyl- und/oder arylsubstituierte Aromaten und substituierte oder unsubstituierte Metallocene. Geeignete alkylsubstituierte Halogenaromaten (Q = Cl, Br, I) sind z. B. 2-Alkyl- oder 4-Alkyl-substituierte Halogenaromaten, wobei Alkyl die bereits vorne definierte Bedeutung besitzen kann und insbesondere für C₁-C₈-Alkyl und besonders bevorzugt für Methyl, Ethyl, Isopropyl und tert-Butyl steht. Besonders bevorzugt sind 2-Methylchlorbenzol, 2-Methylbrombenzol, 4-Methylchlorbenzol, 4-Methylbrombenzol, 2-tert-Butylchlorbenzol, 2-tert-Butylbrombenzol, 4-tert-Butylchlorbenzol und 4-tert-Butylbrombenzol.

Geeignete arylsubstituierte Halogenaromaten (Q = Cl, Br, I) sind z. B. 2-Aryl- oder 4-Arylhalogenaromaten, wobei Aryl die bereits vorne definierte Bedeutung besitzen kann und insbesondere für Phenyl und substituierte Phenylreste und bevorzugt für Phenyl, 2-Tolyl, 4-Tolyl und 2,4-Dimethylphenyl steht. Besonders bevorzugt sind Biphenyl, 9,9-Dimethylxanthen und 4,5-Dibrom-2,7-ditert-butyl- 9,9-dimethylxanthen.

Die Verbindungen der Formel I können gewünschtenfalls isoliert und durch bekannte Methoden, wie z. B. Destillation, Kristallisation, Chromatographie u. ä. gereinigt werden.

Ein bevorzugtes Einsatzgebiet für die erfindungsgemäßen Katalysatoren stellt die Hydroformylierung von Olefinen dar.

Im Allgemeinen werden unter Hydroformylierungsbedingungen aus den jeweils eingesetzten Katalysatoren oder Katalysatorvorstufen katalytisch aktive Spezies der allgemeinen Formel HₓM_{y}(CO)_{z}L_{q} gebildet, worin M für ein Metall der VIII. Nebengruppe, L für einen erfindungsgemäßen Phosphinitliganden und q, x, y, z für ganze Zahlen, abhängig von der Wertigkeit und Art des Metalls sowie der Bindigkeit des Liganden L, stehen. Vorzugsweise stehen z und q unabhängig voneinander mindestens für einen Wert von 1, wie z. B. 1, 2 oder 3. Die Summe aus z und q steht bevorzugt für einen Wert von 2 bis 5. Dabei können die Komplexe gewünschtenfalls zusätzlich noch mindestens einen der zuvor beschriebenen weiteren Liganden aufweisen.

Bei dem Metall M handelt es sich vorzugsweise um Cobalt, Ruthenium, Rhodium, Palladium, Platin, Osmium oder Iridium und insbesondere um Cobalt, Rhodium und Ruthenium.

Nach einer bevorzugten Ausführungsform werden die Hydroformylierungskatalysatoren in situ, in dem für die Hydroformylierungsreaktion eingesetzten Reaktor, hergestellt. Gewünschtenfalls können die erfindungsgemäßen Katalysatoren jedoch auch separat hergestellt und nach üblichen Verfahren isoliert werden. Zur in situ-Herstellung der erfindungsgemäßen Katalysatoren setzt man wenigstens einen Phosphinitliganden der allgemeinen Formel I, eine Verbindung oder einen Komplex eines Metalls der VIII. Nebengruppe, gegebenenfalls wenigstens einen weiteren zusätzlichen Liganden und gegebenenfalls ein Aktivierungsmittel in einem inerten Lösungsmittel unter den Hydroformylierungsbedingungen um.

Geeignete Rhodiumverbindungen oder -komplexe sind z. B. Rhodium(II)- und Rhodium(III)-salze, wie Rhodium(III)-chlorid, Rhodium(III)-nitrat, Rhodium(III)-sulfat, Kalium-Rhodiumsulfat, Rhodium(II)- bzw. Rhodium(III)-carboxylat, Rhodium(II)- und Rhodium(III)-acetat, Rhodium(III)-oxid, Salze der Rhodium(III)-säure, Trisammoniumhexachlororhodat(III) etc. Weiterhin eignen sich Rhodiumkomplexe, wie Rhodiumbiscarbonylacetylacetonat, Acetylacetonatobisethylenrhodium(I) etc. Vorzugsweise werden Rhodiumbiscarbonylacetylacetonat oder Rhodiumacetat eingesetzt.

Ebenfalls geeignet sind Rutheniumsalze oder -verbindungen. Geeignete Rutheniumsalze sind beispielsweise Ruthenium(III)chlorid, Ruthenium(IV)-, Ruthenium(VI)- oder Ruthenium(VIII)oxid, Alkalisalze der Rutheniumsauerstoffsäuren wie K₂RuO₄ oder KRuO₄ oder Komplexverbindungen der allgemeinen Formel RuX¹X²L¹L²(L³)ₙ, worin L¹, L², L³ und n die oben angegebenen Bedeutungen und X¹, X² die für x (siehe oben) angegebenen Bedeutungen besitzen, z. B. RuHCl(CO)(PPh₃)₃. Auch können die Metallcarbonyle des Rutheniums wie Trisrutheniumdodecacarbonyl oder Hexarutheniumoctadecacarbonyl, oder Mischformen, in denen CO teilweise durch Liganden der Formel PR₃ ersetzt sind, wie Ru(CO)₃(PPh₃)₂, im erfindungsgemäßen Verfahren verwendet werden.

Geeignete Cobaltverbindungen sind beispielsweise Cobalt(II)chlorid, Cobalt(II)sulfat, Cobalt(II)carbonat, Cobalt(II)nitrat, deren Amin- oder Hydratkomplexe, Cobaltcarboxylate, wie Cobaltacetat, Cobaltethylhexanoat, Cobaltnaphthoat, sowie der Cobalt-Caprolactamat-Komplex. Auch hier können die Carbonylkomplexe des Cobalts wie Dicobaltoctacarbonyl, Tetracobaltdodecacarbonyl und Hexacobalthexadecacarbonyl eingesetzt werden.

Die genannten Verbindungen des Cobalts, Rhodiums und Rutheniums sind im Prinzip bekannt und in der Literatur hinreichend beschrieben oder sie können vom Fachmann analog zu den bereits bekannten Verbindungen hergestellt werden.

Geeignete Aktivierungsmittel sind z. B. Brönsted-Säuren, Lewis-Säuren, wie z. B. BF₃, AlCl₃, ZnCl₂, und Lewis-Basen.

Als Lösungsmittel werden vorzugsweise die Aldehyde eingesetzt, die bei der Hydroformylierung der jeweiligen Olefine entstehen, sowie deren höher siedende Folgereaktionsprodukte, z. B. die Produkte der Aldolkondensation. Ebenfalls geeignete Lösungsmittel sind Aromaten, wie Toluol, Kohlenwasserstoffe oder Gemische von Kohlenwasserstoffen, auch zum Verdünnen der oben genannten Aldehyde und der Folgeprodukte der Aldehyde. Bei ausreichend hydrophilisierten Liganden können auch Wasser, Alkohole, wie Methanol, Ethanol, n-Propanol, Isopropanol, n-Butanol, Isobutanol, Ketone, wie Aceton und Methylethylketon etc., eingesetzt werden.

Das Molmengenverhältnis von Phosphinitligand der allgemeinen Formel I zu Metall der VIII. Nebengruppe liegt im Allgemeinen in einem Bereich von etwa 1:1 bis 1 000:1.

Ein weiterer Gegenstand der Erfindung ist ein Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart wenigstens eines der erfindungsgemäßen Hydroformylierungskatalysatoren.

Als Substrate für das erfindungsgemäße Hydroformylierungsverfahren kommen prinzipiell alle Verbindungen in Betracht, welche eine oder mehrere ethylenisch ungesättigte Doppelbindungen enthalten. Dazu zählen z. B. Olefine, wie α-Olefine, interne geradkettige und interne verzweigte Olefine. Geeignete α-Olefine sind z. B. Ethylen, Propen, 1-Buten, 1-Penten, 1-Hexen, 1-Hepten, 1-Octen, 1-Nonen, 1-Decen, 1-Undecen, 1-Dodecen etc.

Geeignete geradkettige interne Olefine sind vorzugsweise C₄- bis C₂₀-Olefine, wie 2-Buten, 2-Penten, 2-Hexen, 3-Hexen, 2-Hepten, 3-Hepten, 2-Octen, 3-Octen, 4-Octen etc.

Geeignete verzweigte, interne Olefine sind vorzugsweise C₄- bis C₂₀-Olefine, wie 2-Methyl-2-Buten, 2-Methyl-2-Penten, 3-Methyl-2-Penten, verzweigte, interne Hepten-Gemische, verzweigte, interne Octen-Gemische, verzweigte, interne Nonen-Gemische, verzweigte, interne Decen-Gemische, verzweigte, interne Undecen-Gemische, verzweigte, interne Dodecen-Gemische etc.

Geeignete zu hydroformylierende Olefine sind weiterhin C₅- bis C₈-Cycloalkene, wie Cyclopenten, Cyclohexen, Cyclohepten, Cycloocten und deren Derivate, wie z. B. deren C₁- bis C₂₀-Alkylderivate mit 1 bis 5 Alkylsubstituenten. Geeignete zu hydroformylierende Olefine sind weiterhin Vinylaromaten, wie Styrol, α-Methylstyrol, 4-Isobutylstyrol etc. Geeignete zu hydroformylierende Olefine sind weiterhin α,β-ethylenisch ungesättigte Mono- und/oder Dicarbonsäuren, deren Ester, Halbester und Amide, wie Acrylsäure, Methacrylsäure, Maleinsäure, Fumarsäure, Crotonsäure, Itaconsäure, 3-Pentensäuremethylester, 4-Pentensäuremethylester, Ölsäuremethylester, Acrylsäuremethylester, Methacrylsäuremethylester, ungesättigte Nitrile, wie 3-Pentennitril, 4-Pentennitril, Acrylnitril, vinylether, wie Vinylmethylether, vinylethylether, Vinylpropylether etc., C₁- bis C₂₀-Alkenole, -Alkendiole und -Alkadienole; wie 2,7-Octadienol-1. Geeignete Substrate sind weiterhin Di- oder Polyene mit isolierten oder konjugierten Doppelbindungen. Dazu zählen z. B. 1,3-Butadien, 1,4-Pentadien, 1,5-Hexadien, 1,6-Heptadien, 1,7-Octadien, Vinylcyclohexen, Dicyclopentadien, 1,5,9-Cyclododecatrien sowie Butadienhomo- und -copolymere.

Die Hydroformylierungsreaktion kann kontinuierlich, semikontinuierlich oder diskontinuierlich erfolgen.

Geeignete Reaktoren für die kontinuierliche Umsetzung sind dem Fachmann bekannt und werden z. B. in Ullmanns Enzyklopädie der technischen Chemie, Bd. 1, 3, Aufl., 1951, S. 743 ff. beschrieben.

Geeignete druckfeste Reaktoren sind dem Fachmann ebenfalls bekannt und werden z. B. in Ullmanns Enzyklopädie der technischen Chemie, Bd. 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann.

Die Zusammensetzung des im erfindungsgemäßen Verfahren eingesetzten Synthesegases aus Kohlenmonoxid und Wasserstoff kann in weiten Bereichen variieren. Das molare Verhältnis von Kohlenmonoxid und Wasserstoff beträgt in der Regel etwa 5:95 bis 70:30, bevorzugt etwa 40:60 bis 60:40. Insbesondere bevorzugt wird ein molares Verhältnis von Kohlenmonoxid und Wasserstoff im Bereich von etwa 1:1 eingesetzt.

Die Temperatur bei der Hydroformylierungsreaktion liegt im Allgemeinen in einem Bereich von etwa 20 bis 180 °C, bevorzugt etwa 50 bis 150 °C. Die Reaktion wird in der Regel bei dem Partialdruck des Reaktionsgases bei der gewählten Reaktionstemperatur durchgeführt. Im Allgemeinen liegt der Druck in einem Bereich von etwa 1 bis 700 bar, bevorzugt 1 bis 600 bar, insbesondere 1 bis 300 bar. Der Reaktionsdruck kann in Abhängigkeit von der Aktivität des eingesetzten erfindungsgemäßen Hydroformylierungskatalysators variiert werden. Im Allgemeinen erlauben die erfindungsgemäßen Katalysatoren auf Basis von Phosphinitliganden eine Umsetzung in einem Bereich niedriger Drücke, wie etwa im Bereich von 1 bis 100 bar.

Die erfindungsgemäßen Hydroformylierungskatalysatoren lassen sich nach üblichen, dem Fachmann bekannten Verfahren vom Austrag der Hydroformylierungsreaktion abtrennen und können im Allgemeinen erneut für die Hydroformylierung eingesetzt werden.

Vorteilhafterweise zeigen die erfindungsgemäßen Katalysatoren eine hohe Aktivität, so dass in der Regel die entsprechenden Aldehyde in guten Ausbeuten erhalten werden. Bei der Hydroformylierung von α-Olefinen sowie von innenständigen, linearen Olefinen zeigen sie zudem eine sehr geringe Selektivität zum Hydrierprodukt des eingesetzten Olefins.

Vorzugsweise eignet sich das erfindungsgemäße Hydroformylierungsverfahren zur Herstellung eines Gemischs isomerer Aldehyde, wobei man zur Hydroformylierung eine Verbindung einsetzt, die mindestens eine endständige Doppelbindung enthält, das dadurch gekennzeichnet ist, dass man ein Gemisch isomerer Aldehyde erhält, welches ein n/iso-Verhältnis von mindestens 3:1, insbesondere von 5:1, besonders bevorzugt von 8:1, aufweist.

Die zuvor beschriebenen, erfindungsgemäßen Katalysatoren, die chirale Phosphinitliganden der Formel I umfassen, eignen sich zur enantioselektiven Hydroformylierung.

Die Erfindung wird anhand der folgenden, nicht einschränkenden Beispiele näher erläutert.

### Beispiel

### Niederdruck-Hydroformylierung von 1-Octen

### Beispiel 1:

In einen 50 ml-Glasautoklav wurden 2,6 mg Rhodiumbiscarbonylacetylacetonat (0,01 mmol), 80 mg des erfindungsgemäßen Liganden I.a (R⁹, R¹⁰ = CF₃, 0,20 mmol), 4,5 g 1-Octen (40 mmol) und 5 ml Texanol® bei 100 °C mit einem Synthesegasgemisch CO/H₂ (1:1) bei 10 bar umgesetzt. Nach einer Reaktionszeit von 4 Stunden wurde der Autoklav entspannt und entleert. Das Gemisch wurde mittels GC mit internem Standard analysiert. Der Umsatz betrug 99 %. Die Ausbeute an Nonanal-Isomeren betrug 90 %, die Selektivität bezüglich Nonanal-Isomeren 90 % (90,8 % n-Nonanal, 9,1 % Methyloctanal, 0,1 % Ethylheptanal).

### Vergleichsbeispiel 1

In einen 50 ml-Glasautoklav wurden 2,6 mg Rhodiumbiscarbonylacetylacetonat (0,01 mmol), 262 mg Triphenylphosphin(1,0 mmol), 10,0 g 1-Octen (90 mmol) und 10 ml Texanol® bei 100 °C mit einem Synthesegasgemisch CO/H₂ (1:1) bei 10 bar umgesetzt. Nach einer Reaktionszeit von 4 Stunden wurde der Autoklav entspannt und entleert. Das Gemisch wurde mittels GC mit internem Standard analysiert. Der Umsatz betrug 100 %. Die Ausbeute an Nonanal-Isomeren betrug 88 %, die Selektivität zu Nonanal-Isomeren 89 % (73 % n-Nonanal, 25 % 2-Methyloctanal, 2 % Ethylheptanal).

## Patentansprüche

1. Katalysator, umfassend wenigstens einen Komplex eines Metalls der VIII. Nebengruppe mit mindestens einem ein-, zwei- oder mehrzähnigen Phosphinitliganden der allgemeinen Formel I 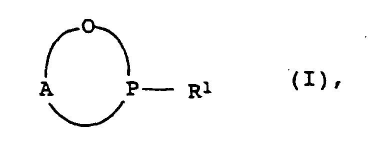 worin
A für einen 2,2'-Biphenylylen-, 2,2'-Binaphthylylen- oder 2,3-Xylylen-Rest steht, der 1, 2 oder 3 Substituenten, ausgewählt unter Alkyl, Alkoxy oder Halogen, tragen kann,
R¹ für Alkyl, Aryl oder Hetaryl steht, welche gegebenenfalls einen, zwei oder drei Substituenten ausgewählt unter Alkyl, Cycloalkyl, Aryl, Alkoxy, Cycloalkyloxy, Aryloxy, Halogen, Trifluormethyl, Nitro, Cyano, Carboxyl, Carboxylat, Acyl, Sulfonyl, NE¹E² und Alkylen-NE¹E² tragen, wobei E¹ und E² gleich oder verschieden sind und ausgewählt sind unter Alkyl, Cycloalkyl und Aryl; oder für einen Rest der allgemeinen Formel II
-X-Y (II)
steht, worin
X entweder für eine C₂- bis C₈-Alkylenbrücke steht, die gegebenenfalls durch SiR^{a}R^{b} , N, NR^{c}, O oder S unterbrochen ist, worin R^{a} und R^{b} für Wasserstoff, Alkyl, Alkoxy, Halogen, Trifluormethyl, Nitro oder Cyano stehen und R^{c} für Wasserstoff, Alkyl oder Aryl steht, wobei Aryl gegebenenfalls durch Alkyl, Alkoxy, Halogen, Trifluormethyl, Nitro oder Cyano ein- oder zweifach substituiert ist, gegebenenfalls eine, zwei oder drei Doppelbindungen aufweist und/oder gegebenenfalls mit ein-, zwei- oder dreifach mit Aryl und/oder Hetaryl anelliert ist, wobei die Aryl- oder Hetarylgruppen unabhängig voneinander gegebenenfalls einen, zwei, drei oder vier Substituenten, ausgewählt unter Alkyl, Cycloalkyl, Aryl, Alkoxy, Cycloalkyloxy, Aryloxy, Halogen, Trifluormethyl, Nitro, Cyano, Acyl, Carboxyl, Carboxylat, NE¹E² oder Alkylen-NE¹E² tragen, wobei E¹ und E² die zuvor angegebenen Bedeutungen besitzen, und benachbarte anellierte Ringe gegebenenfalls über SiR^{a}R^{b} , N, NR^{c}, O oder S oder über eine C₁-C₆-Alkylenbrücke, die gegebenenfalls zusätzlich eine Gruppe, ausgewählt unter SiR^{a}R^{b} , N, NR^{c}, O oder S, trägt, verbunden sind, worin R^{a}, R^{b} und R^{c} wie o. a. definiert sind; oder
für eine gegebenenfalls substituierte Metallocenbrücke steht; und
Y für einen Rest der Formel III 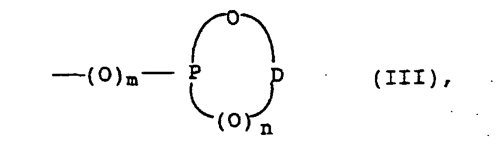 steht, worin
D die zuvor für A angegebenen Bedeutungen besitzt,und
m und n unabhängig voneinander für 0 oder 1 stehen,
oder ein Salz davon.

2. Katalysator nach Anspruch 1, worin X für einen Rest der Formel IV.1, IV.2, IV.3, IV.4, IV.5, IV.6, IV.7 oder IV.8 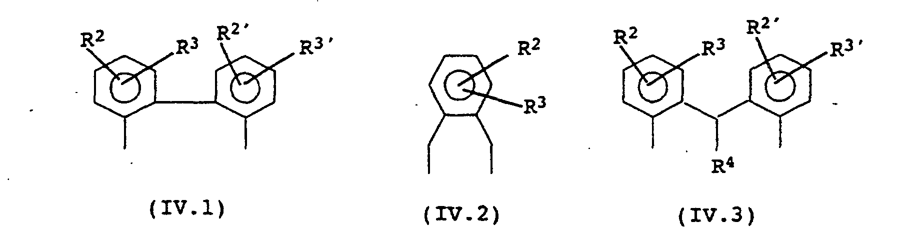 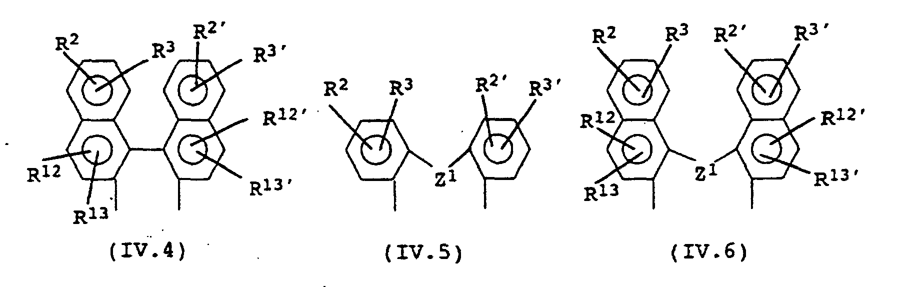 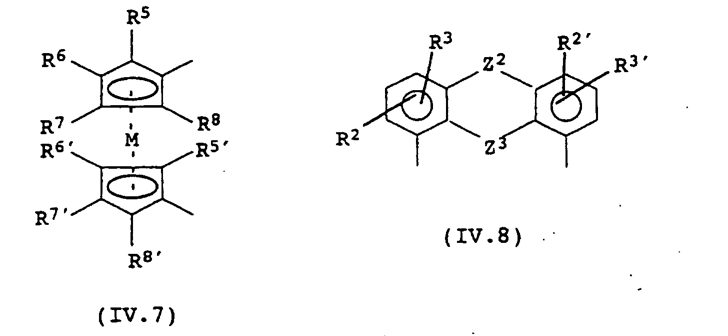 steht, worin
R² , R^{2'}, R³ und R^{3'} unabhängig voneinander für Wasserstoff, Alkyl, Alkoxy, Halogen, Trifluormethyl, Nitro, Cyano, Acyl, Carboxyl oder Carboxylat stehen,
R⁴ für Wasserstoff, Alkyl oder Aryl steht, welches gegebenenfalls durch Alkyl, Alkoxy, Halogen, Trifluormethyl, Nitro oder Cyano substituiert sein kann, und
R⁵, R^{5'}, R⁶, R^{6'}, R⁷, R^{7'}, R⁸, R^{8'}, R¹², R^{12'}, R¹³ und R^{13'} die für R² und R³ angegebenen Bedeutungen besitzen oder unabhängig voneinander ausgewählt sind unter Cycloalkyl, Aryl oder Alkyl, die durch ein Sauerstoffatom unterbrochen oder mit einem Rest der Formel NE¹E² substituiert sein können, wobei E¹ und E² die oben angegebene Bedeutung besitzen; oder jeweils zwei benachbarte Substituenten R⁵, R⁶, R⁷, R⁸ und/oder R^{5'}, R^{6'}, R^{7'}, R^{8'} mit dem sie verbindenden Teil des Cyclopentadienylrings für einen aromatischen oder nichtaromatischen 5- bis 7-gliedrigen Carbooder Heterocyclus stehen, wobei der Heterocyclus 1, 2 oder 3 Heterogruppen, ausgewählt unter O, N, NR^{c} und S, aufweist,
M für Fe, Co, Ni, Ru, Os, Rh, Mn, Cr oder V steht,
Z¹ für O, S und NR^{c} oder für eine C₂- bis C₃-Alkylenbrücke, die durch O, S oder NR^{c} unterbrochen ist, steht,
Z² und Z³ unabhängig voneinander für CH₂, SiR^{a}R^{b}, O, S, NR^{c} oder CR^{a}R^{b} stehen, wobei R^{a}, R^{b} und R^{c} jeweils die oben angegebene Bedeutung besitzen.

3. Katalysator nach einem der vorhergehenden Ansprüche, wobei der Phosphinitligand der Formel I ausgewählt ist unter Liganden der Formeln Ia bis Ii 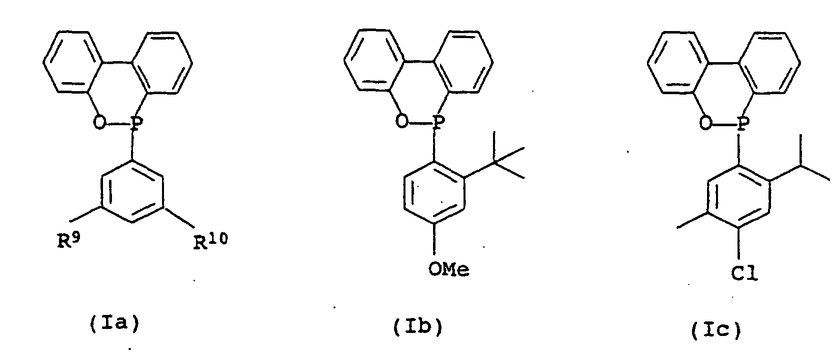 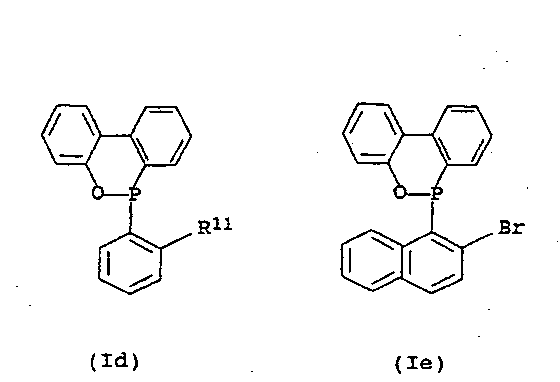 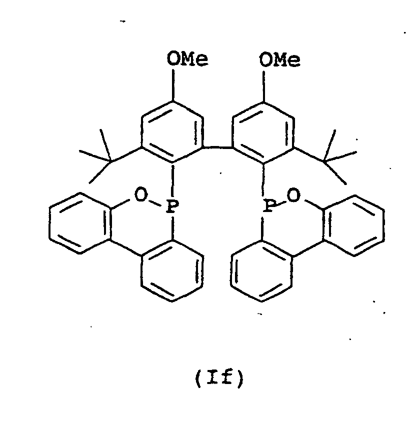 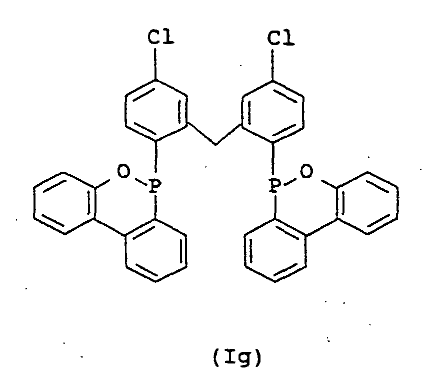 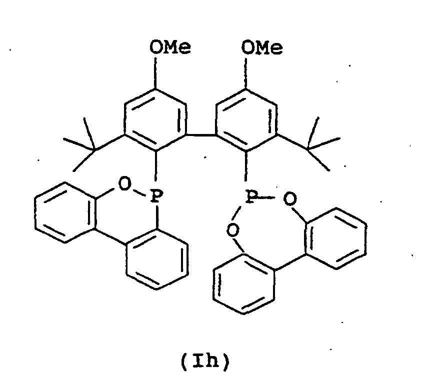 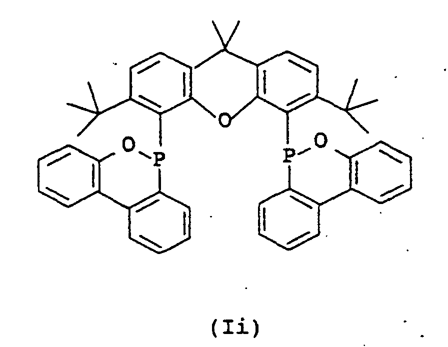 worin
R⁹ und R¹⁰ unabhängig voneinander für Wasserstoff oder Trifluormethyl stehen und
R¹¹ für Fluor oder Trifluormethyl steht.

4. Katalysator nach einem der vorhergehenden Ansprüche, **dadurch gekennzeichnet, dass** das Metall der VIII. Nebengruppe ausgewählt ist unter Cobalt, Nickel, Ruthenium, Rhodium, Palladium und Platin.

5. Katalysator nach einem der vorhergehenden Ansprüche, der zusätzlich wenigstens einen weiteren von Liganden der Formel I verschiedenen Liganden, ausgewählt unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃ sowie ein-, zwei- und mehrzähnigen Phosphin-, Phosphinit-, Phosphonit- und Phosphitliganden aufweist.

6. Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart eines Hydroformylierungskatalysators, **dadurch gekennzeichnet, dass** man als Hydroformylierungskatalysator einen Katalysator nach einem der Ansprüche 1 bis 5 einsetzt.

7. Verfahren nach Anspruch 6, wobei man zur Hydroformylierung eine Verbindung einsetzt, die mindestens eine endständige Doppelbindung enthält, **dadurch gekennzeichnet, dass** man ein Gemisch isomerer Aldehyde mit einem n/iso-Verhältnis von mindestens 3:1, insbesondere 5:1, besonders bevorzugt 8:1, erhält.

8. Verfahren nach Anspruch 6, **dadurch gekennzeichnet, dass** der Hydroformylierungskatalysator in situ hergestellt wird, wobei man mindestens einen Phosphinitliganden der allgemeinen Formel I, wie in den Ansprüchen 1 bis 5 definiert, eine Verbindung oder einen Komplex eines Metalls der VIII. Nebengruppe und gegebenenfalls ein Aktivierungsmittel in einem inerten Lösungsmittel unter den Hydroformylierungs bedingungen zur Reaktion bringt.

9. Verwendung von Katalysatoren, umfassend einen Phosphinitliganden der Formel I, gemäß einem der Ansprüche 1 bis 5 zur Hydroformylierung von Verbindungen mit wenigstens einer ethylenisch ungesättigten Doppelbindung.

## Claims

1. A catalyst comprising at least one complex of a metal of group VIII with at least one uni-, bi- or multidentate phosphinite ligand of the general formula I 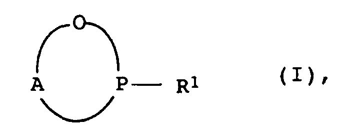 in which
A is a 2,2'-biphenylylene, 2,2'-binaphthylylene or 2,3-xylylene radical, which may have 1, 2 or 3 substituents selected from alkyl, alkoxy or halogen,
R¹ is alkyl, aryl or hetaryl, each of which optionally has one, two or three substituents selected from alkyl, cycloalkyl, aryl, alkoxy, cycloalkyloxy, aryloxy, halogen, trifluoromethyl, nitro, cyano, carboxyl, carboxylate, acyl, sulfonyl, NE¹E² and alkylene-NE¹E^{2,} where E¹ and E² are identical or different and are selected from alkyl, cycloalkyl and aryl; or is a radical of the general formula II
-X-Y (II)
in which
X is either a C₂-C₈-alkylene bridge which is optionally interrupted by SiR^{a}R^{b}, N, NR^{c}, O or S, in which R^{a} and R^{b} are hydrogen, alkyl, alkoxy, halogen, trifluoromethyl, nitro or cyano, and R^{c} is hydrogen, alkyl or aryl, where aryl is optionally mono- or disubstituted by alkyl, alkoxy, halogen, trifluoromethyl, nitro or cyano, optionally has one, two or three double bonds and/or is optionally fused once, twice or three times to aryl and/or hetaryl, where the aryl or hetaryl groups have, independently of one another, optionally one, two, three or four substituents selected from alkyl, cycloalkyl, aryl, alkoxy, cycloalkyloxy, aryloxy, halogen, trifluoromethyl, nitro, cyano, acyl, carboxyl, carboxylate, NE¹E² and alkylene-NE¹E^{2,} where E¹ and E² have the meanings indicated previously, and adjacent fused rings are optionally connected by SiR^{a}R^{b}, N, NR^{c}, O or S or by a C₁-C₆-alkylene bridge which optionally additionally carries a group selected from SiR^{a}R^{b}, N, NR^{c}, O or S, in which R^{a}, R^{b} and R^{c} are as defined above; or
an optionally substituted metallocene bridge; and
Y is a radical of the formula III 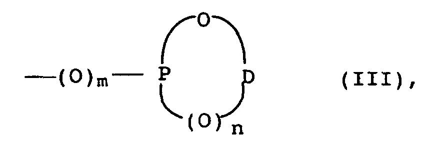 in which
D has the meanings indicated previously for A, and
m and n are, independently of one another, 0 or 1,
or a salt thereof.

2. A catalyst as claimed in claim 1, in which X is a radical of the formula IV.1, IV.2, IV.3, IV.4, IV.5, IV.6, IV.7 or IV.8 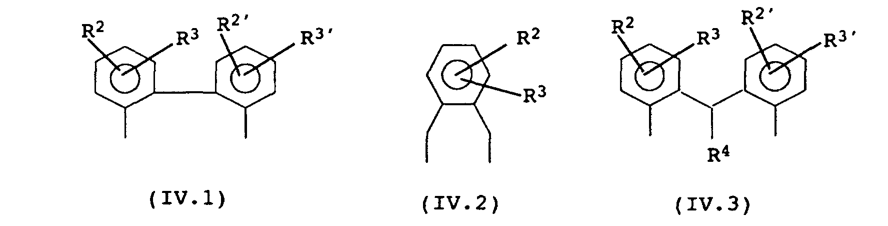 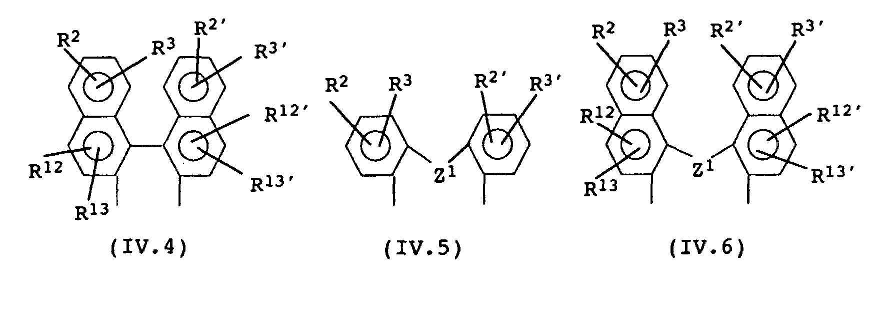 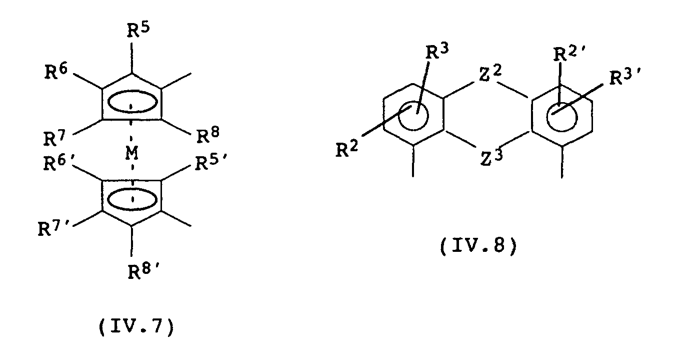 in which
R² , R^{2'}, R³ and R^{3'} are, independently of one another, hydrogen, alkyl, alkoxy, halogen, trifluoromethyl, nitro, cyano, acyl, carboxyl or carboxylate,
R⁴ is hydrogen, alkyl, or aryl, which may optionally be substituted by alkyl, alkoxy, halogen, trifluoromethyl, nitro or cyano, and
R⁵, R^{5'}, R⁶, R^{6'}, R⁷, R^{7'}, R⁸, R^{8'}, R¹², R^{12'}, R¹³ and R^{13'} have the meanings mentioned for R² and R³ or are, independently of one another, selected from cycloalkyl, aryl or alkyl, which may be interrupted by an oxygen atom or substituted by a radical of the formula NE¹E², where E¹ and E² have the meaning indicated above; or in each case two adjacent substituents R⁵, R⁶, R⁷, R⁸ and/or R^{5'}, R^{6'}, R^{7'}, R^{8'} represent, with the part of the cyclopentadienyl ring connecting them, an aromatic or nonaromatic 5- to 7-membered carbocycle or heterocycle, where the heterocycle has 1, 2 or 3 heterogroups selected from O, N, NR^{c} and S,
M is Fe, Co, Ni, Ru, Os, Rh, Mn, Cr or V,
Z¹ is O, S and NR^{c} or a C₂- to C₃-alkylene bridge which is interrupted by O, S or NR^{c},
Z² and Z³ are, independently of one another, CH₂, SiR^{a}R^{b}, O, S, NR^{c} or CR^{a}R^{b}, where R^{a}, R^{b} and R^{c} each have the meaning indicated above.

3. A catalyst as claimed in either of the preceding claims, where the phosphinite ligand of the formula I is selected from ligands of the formulae Ia to Ii 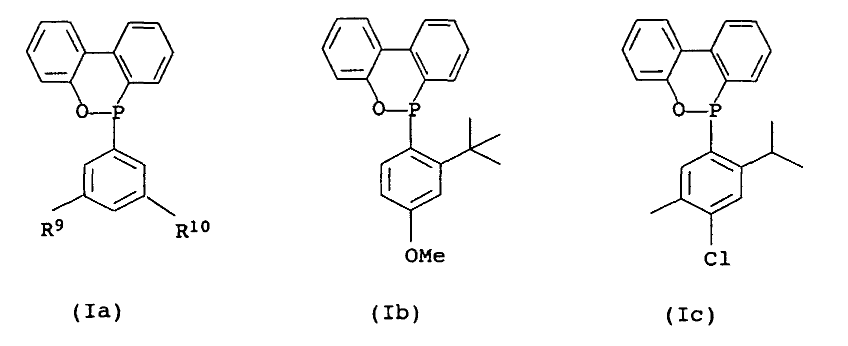 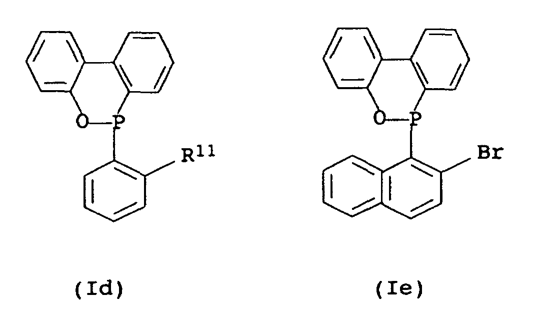 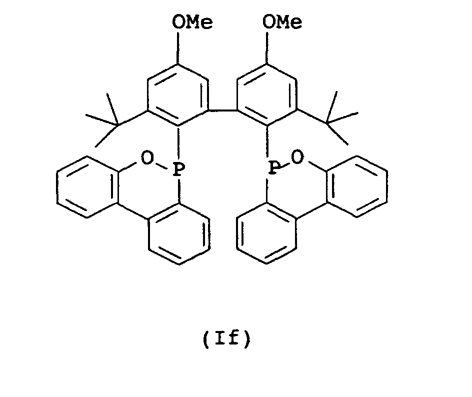 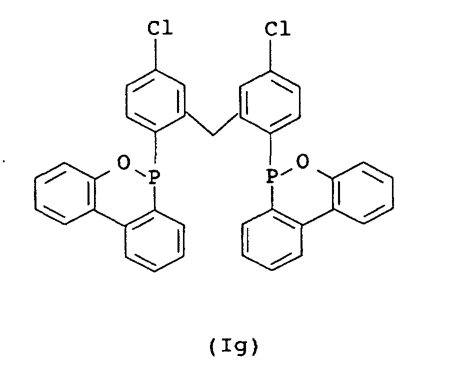 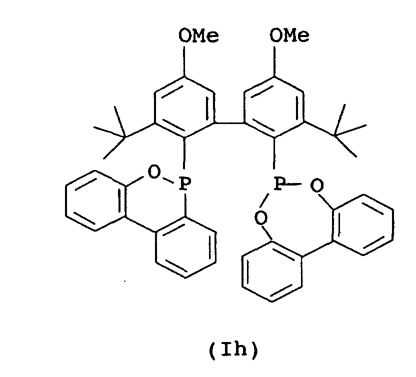 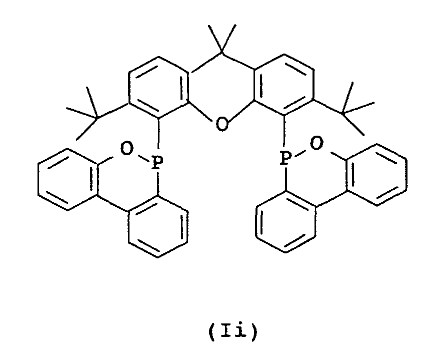 in which
R⁹ and R¹⁰ are, independently of one another, hydrogen or trifluoromethyl, and
R¹¹ is fluorine or trifluoromethyl.

4. A catalyst as claimed in any of the preceding claims, wherein the metal of group VIII is selected from cobalt, nickel, ruthenium, rhodium, palladium and platinum.

5. A catalyst as claimed in any of the preceding claims, which additionally comprises at least one other ligand different from ligands of the formula I and selected from halides, amines, carboxylates, acetylacetonate, aryl- or alkylsulfonates, hydrides, CO, olefins, dienes, cycloolefins, nitriles, N-containing heterocycles, aromatic and heteroaromatic compounds, ethers, PF₃, and uni-, bi- and multidentate phosphine, phosphinite, phosphonite and phosphite ligands.

6. A process for the hydroformylation of compounds which contain at least one ethylenic double bond, by reaction with carbon monoxide and hydrogen in the presence of a hydroformylation catalyst, wherein a catalyst as claimed in any of claims 1 to 5 is employed as hydroformylation catalyst.

7. A process as claimed in claim 6, employing for the hydroformylation a compound which contains at least one terminal double bond, wherein a mixture of isomeric aldehydes with an n/iso ratio of at least 3:1, in particular 5:1, particularly preferably 8:1, is obtained.

8. A process as claimed in claim 6, wherein the hydroformylation catalyst is prepared in situ, with at least one phosphinite ligand of the general formula I as defined in claims 1 to 5, a compound or a complex of a metal of group VIII and, where appropriate, an activator being reacted in an inert solvent under the hydroformylation conditions.

9. The use of catalysts comprising a phosphinite ligand of the formula I as claimed in any of claims 1 to 5 for the hydroformylation of compounds having at least one ethylenic double bond.

## Revendications

1. Catalyseur comprenant au moins un complexe d'un métal du groupe VIIIB avec au moins un ligand phosphinite mono-, bi- ou multidenté de formule générale I 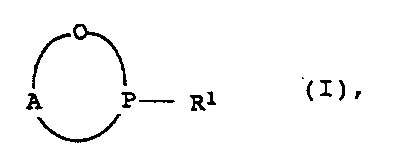 dans laquelle
A représente un radical 2,2'-biphénylylène, 2,2'-binaphtylylène ou 2,3-xylylène, qui peut porter 1, 2 ou 3 substituants choisis parmi des atomes d'halogène et des groupes alkyle et alcoxy,
R¹ représente un groupe alkyle, aryle ou hétéroaryle portant éventuellement un, deux ou trois substituants choisis parmi des atomes d'halogène et des groupes alkyle, cycloalkyle, aryle, alcoxy, cycloalkyloxy, aryloxy, trifluorométhyle, nitro, cyano, carboxy, carboxylate, acyle, sulfonyle, NE¹E² et alkylène-NE¹E², E¹ et E² étant identiques ou différents et étant choisis parmi des groupes alkyle, cycloalkyle et aryle ; ou représente un radical de formule générale II
-X-Y (II)
dans laquelle
X représente soit un pont alkylène en C₂-C₈ qui est éventuellement interrompu par SiR^{a}R^{b}, N, NR^{c}, O ou S, où R^{a} et R^{b} représentent un atome d'hydrogène ou d'halogène ou un groupe alkyle, alcoxy, trifluorométhyle, nitro ou cyano et R^{c} représente un atome d'hydrogène ou un groupe alkyle ou aryle, le groupe aryle étant éventuellement une ou deux fois substitué un ou des atomes d'halogène ou groupes alkyle, alcoxy, trifluorométhyle, nitro ou cyano, comporte éventuellement une, deux ou trois doubles liaisons et/ou est éventuellement soudé une, deux ou trois fois à un groupe aryle et/ou hétéroaryle, les groupes aryle ou hétéroaryle, indépendamment les uns des autres, portant éventuellement un, deux, trois ou quatre substituants choisis parmi des atomes d'halogène et des groupes alkyle, cycloalkyle, aryle, alcoxy, cycloalkyloxy, aryloxy, trifluorométhyle, nitro, cyano, acyle, carboxy, carboxylate, NE¹E² ou alkylène-NE¹E², E¹ et E² ayant les significations indiquées précédemment, et des cycles condensés voisins étant éventuellement reliés par SiR^{a}R^{b}, N, NR^{c}, O ou S ou par un pont alkylène en C₁-C₆ qui éventuellement porte additionnellement un groupe choisi parmi SiR^{a}R^{b}, N, NR^{c}, O et S, R^{a}, R^{b} et R^{c} étant tels que définis plus haut ; ou
représente soit un pont métallocène éventuellement substitué ; et
Y représente un radical de formule III 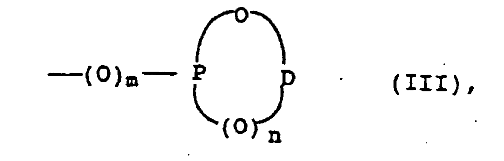 dans laquelle
D a les significations indiquées précédemment pour A, et
m et n représentent, indépendamment l'un de l'autre, 0 ou 1,
ou un sel d'un tel composé.

2. Catalyseur selon la revendication 1, dans lequel X représente un radical de formule IV.1, IV.2, IV.3, IV.4, IV.5, IV.6, IV.7 ou IV.8 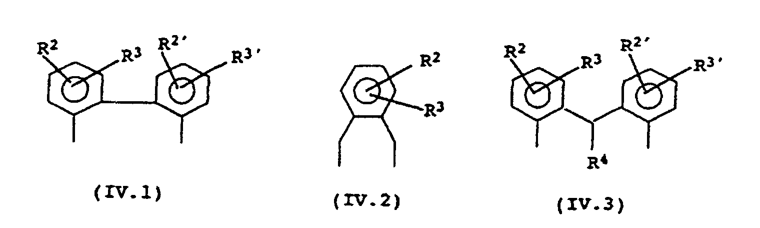 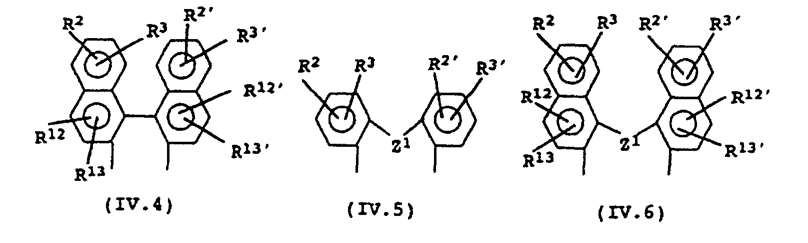 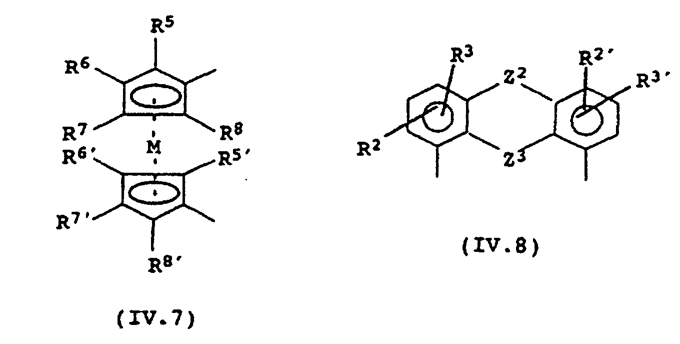 formules dans lesquelles
R², R^{2'}, R³ et R^{3'} représentent, indépendamment les uns des autres, un atome d'hydrogène ou d'halogène ou un groupe alkyle, alcoxy, trifluorométhyle, nitro, cyano, acyle, carboxy ou carboxylate,
R⁴ représente un atome d'hydrogène ou un groupe alkyle ou aryle qui peut éventuellement être substitué par alkyle, alcoxy, halogéno, trifluorométhyle, nitro ou cyano, et
R⁵, R^{5'}, R⁶, R^{6'}, R⁷, R^{7'}, R⁸, R^{8'}, R¹², R^{12'}, R¹³ et R^{13'} ont les significations indiquées pour R² et R³ ou sont choisis, indépendamment les uns des autres, parmi des groupes cycloalkyle, aryle ou alkyle, qui peuvent être interrompus par un atome d'oxygène ou par un radical de formule NE¹E², E¹et E² ayant les significations indiquées plus haut ; ou deux substituants voisins R⁵, R⁶, R⁷, R⁸ et/ou R^{5'}, R^{6'}, R^{7'}, R^{8'} représentent, respectivement, avec la partie du cycle cyclopentadiényle qui les relie, un cycle carbocylique ou hétérocyclique, aromatique ou non aromatique, à 5-7 chaînons, le cycle hétérocyclique comportant 1, 2 ou 3 hétérogroupes choisis parmi O, N, NR^{c} et S,
M représente Fe, Co, Ni, Ru, Os, Rh, Mn, Cr ou V,
Z¹ représente O, S ou NR^{c} ou un pont alkylène en C₂-C₃, qui est interrompu par O, S ou NR^{c},
Z² et Z³ représentent, indépendamment l'un de l'autre, CH₂, SiR^{a}R^{b}, O, S, NR^{c} ou CR^{a}R^{b}, R^{a}, R^{b} et R^{c} ayant chacun la signification indiquée plus haut.

3. Catalyseur selon l'une quelconque des revendications précédentes, dans lequel le ligand phosphinite de formule I est choisi parmi les ligands de formules Ia à Ii 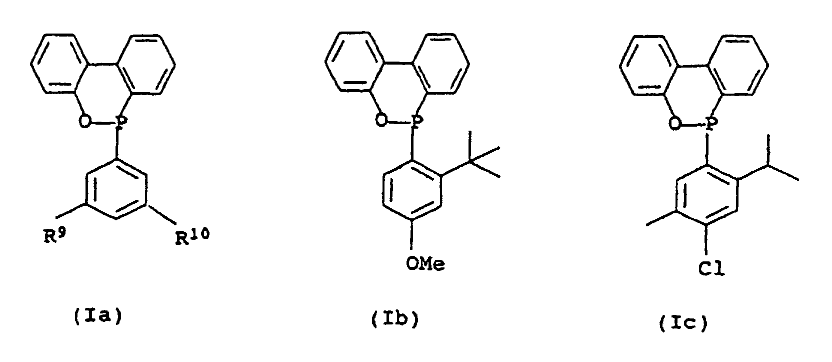 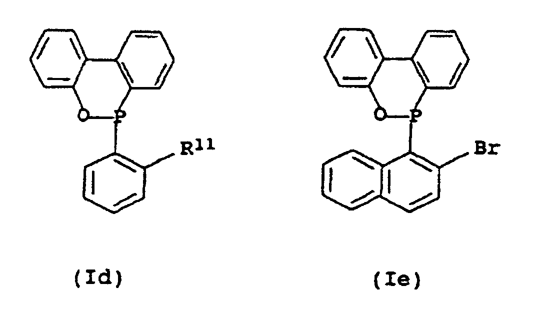 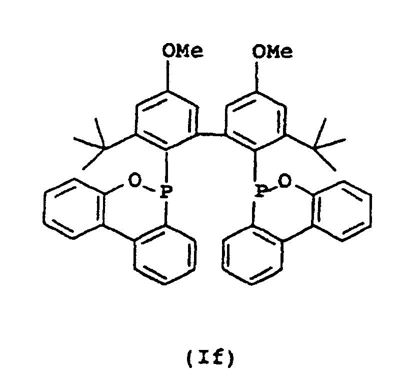 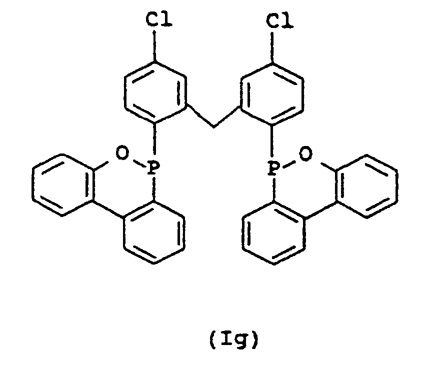 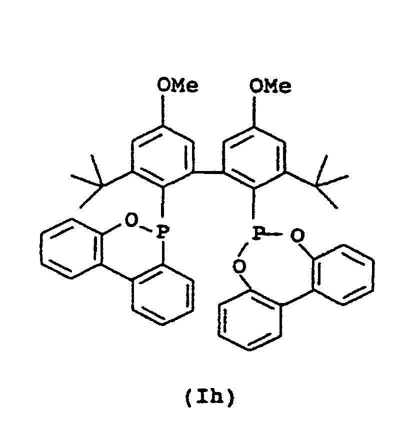 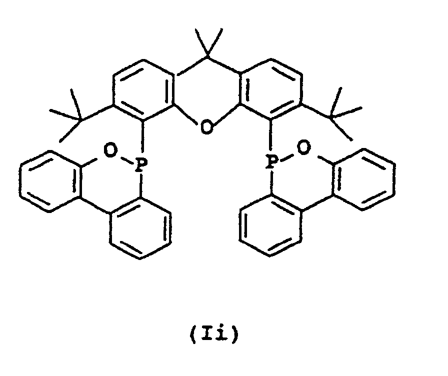 dans lesquelles
R⁹ et R¹⁰ représentent, indépendamment l'un de l'autre, un atome d'hydrogène ou le groupe trifluorométhyle et
R¹¹ représente un atome de fluor ou le groupe trifluorométhyle.

4. Catalyseur selon l'une quelconque des revendications précédentes, **caractérisé en ce que** le métal du groupe VIIIB est choisi parmi le cobalt, le nickel, le ruthénium, le rhodium, le palladium et le platine.

5. Catalyseur selon l'une quelconque des revendications précédente, qui comporte additionnellement au moins un autre ligand différent des ligands de formule I, choisi parmi des halogénures, des amines, des carboxylates, un acétylacétonate, des aryl- ou alkylsulfonates, un hydrure, CO, des oléfines, des diènes, des cyclo-oléfines, des nitriles, des hétérocycles azotés, des composés aromatiques et hétéroaromatiques, des éthers, PF₃ ainsi que des ligands phosphine, phosphinite, phosphonite et phosphite mono-, bi- et multidentés.

6. Procédé pour l'hydroformylation de composés qui comportent au moins une double liaison à insaturation éthylénique, par réaction avec du monoxyde de carbone et de l'hydrogène en présence d'un catalyseur d'hydroformylation, **caractérisé en ce qu'**on utilise comme catalyseur d'hydroformylation un catalyseur selon l'une quelconque des revendications 1 à 5.

7. Procédé selon la revendication 6, dans lequel on utilise dans l'hydroformylation un composé qui comporte au moins une double liaison en bout de chaîne, **caractérisé en ce qu'**on obtient un mélange d'aldéhydes isomères présentant un rapport n/iso d'au moins 3:1, en particulier 5:1, de façon particulièrement préférée 8:1.

8. Procédé selon la revendication 6, **caractérisé en ce que** le catalyseur d'hydroformylation est préparé in situ, en faisant réagir dans les conditions d'hydroformylation au moins un ligand phosphinite de formule générale I, telle que définie dans les revendications 1 à 5, un composé ou un complexe d'un métal du groupe VIIIB et éventuellement un activateur, dans un solvant inerte.

9. Utilisation de catalyseurs, comprenant un ligand phosphinite de formule I, selon l'une quelconque des revendications 1 à 5, pour l'hydroformylation de composés comportant au moins une double liaison à insaturation éthylénique.
